# EUROPEAN PATENT APPLICATION

(11) **EP 4 286 405 A1**
(43) Date of publication of application: **06.12.2023**
(21) Application number: 22746285.0
(22) Date of filing: 28.01.2022
(51) Int. Cl.: C07K 14/47, C12N 9/02, A61K 48/00, A61P 25/28

(54) **GENE THERAPY FOR TREATING NEURODEGENERATIVE DISEASES**

(30) Priority: 28.01.2021 KR 20210012117
(71) Applicant: Abrain, Suwon-si Gyeonggi-do 16209 (KR)
(72) Inventor: PARK, Kyung Won, Suwon-si, Gyeonggi-do 16413 (KR)
(74) Representative: Altmann Stößel Dick Patentanwälte PartG mbB
(86) International application number: PCT/KR2022/001594
(87) International publication number: WO 2022/164259

(57) **Abstract**

The present invention provides a novel gene-therapeutic agent for neurodegenerative diseases.

The present invention allows Aβ variants to be secreted out of cells to allow wt Aβ polymerization to be slowed or inhibited and cytotoxicity to be reduced in the human body, and thus exhibits excellent effects of preventing, alleviating, and treating neurodegenerative diseases.

## Description

### [Technical Field]

The present invention relates to gene therapy for the treatment of neurodegenerative diseases.

### [Background Art]

Neurodegenerative diseases are those that primarily affect neurons. Progressive loss of neuronal structure, progressive loss of neuronal function, or progressive neuronal cell death may be involved in a degenerative process. Several specific disorders are classified as neurodegenerative diseases.

It is reported that Alzheimer's disease accounts for about 60% of all dementia cases, and more than 26 million individuals worldwide have Alzheimer's disease. Dementia usually involves progressive decline in mental function, including deficits in memory, language and cognitive processes. Alzheimer's disease may not only affect the patients themselves, but it also affects the millions of caregivers who have to care for patients, often without being paid. Since the greatest risk factor for Alzheimer's disease is age, the prevalence increases dramatically as people live longer in older age.

Typical pathologies associated with Alzheimer's disease involve macroscopic atropy of the brain, thinning of grey matter of the cerebral cortex, enlarged ventricles suggesting neuronal loss, beta-amyloid peptide [Aβ]-containing microscopic extracellular amyloid plaques aggregated into protein clumps, extracellular neurofibrillary tangles including aggregated tau protein, and cerebrovascular amyloid, i.e., perivascular amyloid protein.

In particular, the pathological features of Alzheimer's disease are the presence of neurofibrillary tangles (NFTs) and amyloid deposits in the patient's brain. Senile plaques are known to be extracellular accumulation of aggregated amyloid-β protein (Aβ), and develop around nerve endings.

Decades of research have been devoted to understanding the processing and accumulation of pathological Aβ in Alzheimer's disease. Efforts to develop anti-Aβ therapies based on this study have focused on inhibiting β-secretase and γ-secretase, isolating peptides released from neurons through immunotherapy, or preventing Aβ polymerization. These therapies have not been very successful since most of the clinical trials testing these approaches have yielded unsatisfactory results. A recent reanalysis of the EMERGE trial for aducanumab immunotherapy has given new hope to the idea that targeting Aβ targeting is clinically feasible. It is unlikely that optimal AD treatment will target Aβ alone, but it may form part of a future combination therapy tailored to the disease stage. Antibody therapy, however, may be the first Aβ-reducing strategy to reach the clinic, but may have problems with widespread use due to the side-effect profile and the need for repeated intravenous administration.

In addition, tremendous efforts have recently been made to develop small peptides capable of preventing the formation of amyloid and toxic oligomers. Peptides provide high biological activity associated with high specificity and low toxicity, but despite these advantages, the efficacy of peptide drugs may be severely hampered by their short half-lives in vivo, and may cause problems especially in administration and delivery to the brain.

Therefore, in view of the foregoing, there is a need for improved gene therapy for the promotion of neuronal regeneration or survival, treating, preventing, or alleviating neurodegenerative disorders.

### [Related-art Documents]

### [Patent Documents]

(Patent Document 0001) Korean Patent Laid-Open Publication No. 10-2020-0075865
(Patent Document 0002) Korean Patent Laid-Open Publication No. 10-2019-0127266

### [Disclosure]

### [Technical Problem]

The present inventors developed a novel expression vector-based gene therapy for secreting Aβ variants in order to overcome the limitations of the related art on BBB penetration and half-life in vivo.

Accordingly, the present invention relates to a genetic construct, a recombinant expression vector, and a use thereof, capable of expressing an Aβ variant that slows down or hinders wt Aβ polymerization and reduces its toxicity in the human body.

### [Technical Solution]

The present inventors constructed a novel genetic construct encoding an Aβ peptide variant.

Accordingly, the present invention provides a coding sequence encoding an Aβ peptide variant; and a promoter operably linked thereto.

The present inventors have confirmed that using the above genetic construct of the present invention, it is possible to achieve gene therapy for neurodegenerative diseases with a small number of administrations (single administration or more) without the need for direct injection of the recombinant protein. In particular, the Aβ peptide variant may interfere with or inhibit wt Aβ, and may exhibit excellent disease improvement and therapeutic effects by the disassembly of the existing aggregated Aβ plaque.

Preferably, the Aβ peptide variant according to the present invention may be a variant based on the amyloid precursor protein sequence. The amyloid precursor protein sequence is known to have three isoforms, APP 695, 751, and 770 aa. Among the isoforms of the amyloid precursor protein, the isoform 695 aa is known to be mainly expressed in nerves. Variants based on the base sequence are included in the scope of the present invention. For example, a representative mRNA APP sequence can be found in GenBank^{®} Accession No. NM_000484.

The Aβ peptide variant according to the present invention may comprise at least any one mutation selected from the group consisting of V689P, F690D and A692D based on the amyloid precursor protein sequence (SEQ ID NO: 1, based on APP 770 aa (nucleic acid sequence: SEQ ID NO: 2)). In other words, the coding sequence encoding the Aβ peptide variant may be a sequence encoding a peptide sequence including any one or more mutations selected from the group consisting of the above-described V689P, F690D, and A692D.

Exemplary sequences of these encoding sequences are specifically described in SEQ ID NOs: 3 or 4, respectively. SEQ ID NOs: 3 or 4 are designed to include V689P/A692D, or V689P/F690D/A692D mutations, respectively, and correspond to examples of arbitrary variants, and the content is not limited to these variants. In other words, those containing any one or more mutations selected from the group consisting of V689P, F690D, and A692D and encoding sequences thereof are included in the scope of the present invention.

The variants according to the present invention may further comprise additional variants thereof to the extent that "biological active" is equally maintained.

Thus, the present invention may comprise additional mutations in the sequence of any one of SEQ ID NOs: 3 or 4. Specifically, a sequence having at least 80%, at least 85%, at least 90%, at least 91%, at least 92%, at least 93%, at least 94%, at least 95%, at least 96%, at least 97%, at least 98%, or at least 99% sequence homology to each sequence may be employed. These sequences are intended to include within the scope of the present invention sequences in which the biological activity of the above Aβ peptide variant produced by the encoding sequence is maintained at an equivalent level. More specifically, sequence modifications may be made or designed in the sequences of SEQ ID NOs: 3 or 4 so as to include additional mutations at positions 689 to 692 of the Aβ peptide. These sequences have at least 80%, at least 85%, at least 90%, at least 91%, at least 92%, at least 93%, at least 94%, at least 95%, at least 96%, at least 97%, at least 98%, or at least 99% sequence homology, and may include within the scope of the present invention sequences in which the biological activity of the produced Aβ peptide variant is maintained at an equivalent level.

Examples of peptide sequences encoded from SEQ ID NOs: 3 or 4 are shown in SEQ ID NOs: 5 or 6, respectively.

The Aβ peptide variant may induce a steric change that interferes with Aβ polymerization through interaction with wt Aβ monomer. In other words, Aβ polymerization may be formed from the central hydrophobic cluster of Aβ, and Aβ peptide variants containing the above-described mutation sequences interact with wt Aβ to inhibit aggregation and reduce toxicity. That is, the aggregation inhibitory action enables the reduction of amyloid formation and Aβ-mediated neurotoxicity. These Aβ peptide variants themselves do not cause aggregation or toxicity, but when present with wt Aβ peptides, the peptide variants may exhibit a therapeutic effect of diseases through suppression of wt Aβ polymerization or disassembly of deposited Aβ.

Aβ peptides are produced as a result of excessive processing of the amyloid precursor protein (APP) which is a parent trans-membrane protein found in neurons and other cells. Amyloid plaques are composed primarily of 40 and 42 amino acid peptides (called Aβ40 and Aβ42, respectively) derived from the amyloid precursor protein (APP) by sequential proteolysis catalyzed by aspartyl protease, which is beta-secretase, and subsequent presenilin-dependent gammasecretase cleavage. Aβ42 peptide is more hydrophobic and less soluble than Aβ40 peptide and is the dominant species within amyloid plaques.

In particular, there is a strong tendency for Aβ42 to aggregate and deposit, which tends to cause more cytotoxicity as well as synaptic loss. Accordingly, an object of the present invention is to provide an Aβ42 peptide variant.

The aggregation and deposition of the Aβ42 peptide have a great impact on neurodegenerative diseases, especially Alzheimer's disease.

Therefore, in the present invention, the Aβ42 peptide variant may contain any one or more mutations selected from the group consisting of V18P, F19D, and A21D based on the Aβ42 sequence (SEQ ID NO: 7 (nucleic acid sequence: SEQ ID NO: 8)) that greatly affects the aggregation and deposition of the above peptides. In other words, the first coding sequence encoding the Aβ peptide variant may be a sequence encoding a peptide sequence including any one or more mutations selected from the group consisting of the above-described V18P, F19D, and A21D.

Exemplary sequences of these encoding sequences are specifically described in SEQ ID NOs: 9 or 10, respectively. SEQ ID NOs: 9 or 10 are designed to include V18P/A21D or V18P/F19D/A21D mutations, respectively, and correspond to examples of arbitrary variants, and the content is not limited to these variants. That is, those containing one or more mutations selected from the group consisting of V18P, F19D, and A21D and encoding sequences thereof are included in the scope of the present invention.

The variants according to the present invention may further comprise additional mutations thereof to the extent that "biological activity" is equally maintained.

Therefore, the present invention may comprise additional mutations in the sequence of any one of SEQ ID NOs: 9 or 10. Specifically, a sequence having at least 80%, at least 85%, at least 90%, at least 91%, at least 92%, at least 93%, at least 94%, at least 95%, at least 96%, at least 97%, at least 98%, or at least 99% sequence homology to each sequence may be employed. These sequences are intended to include within the scope of the present invention sequences in which the biological activity of the above Aβ42 peptide variant produced by the encoding sequence is maintained at an equivalent level.

More specifically, sequence modifications may be made or designed in the sequences of SEQ ID NOs: 9 or 10 so as to include additional mutations at positions 18 to 21 of the Aβ42 peptide. These sequences have at least 80%, at least 85%, at least 90%, at least 91%, at least 92%, at least 93%, at least 94%, at least 95%, at least 96%, at least 97%, at least 98%, or at least 99% sequence homology, and may include within the scope of the present invention sequences in which the biological activity of the produced Aβ42 peptide variant is maintained at an equivalent level.

According to specific embodiments according to the present invention, the Aβ42 peptide variant may be any one or more selected from the group consisting of the following sequences, but is not limited thereto:
Aβ(V18P/A21D) :
   DAEFRHDSGYEVHHQKLPFFDEDVGSNKGAIIGLMVGGVVIA (SEQ ID NO: 11)
Aβ(V18P/F19D/A21D) :
   DAEFRHDSGYEVHHQKLPDFDEDVGSNKGAIIGLMVGGVVIA (SEQ ID NO: 12)

As described above, the present invention includes all of each mutation of V18P, F19D, or A21D, two combined mutations, and three combined mutations thereof. If desired, sequences having at least 95%, at least 96%, at least 97%, at least 98%, or at least 99% sequence homology to SEQ ID NOs: 11 or 12 while including the above-described mutations, may also be included in the scope of the present invention.

In the present invention, the Aβ peptide variant and/or the Aβ42 peptide variant may further comprise a sequence of TVIVITLVMLKK (SEQ ID NO: 13) at the end of the sequence (represented by the abbreviation KK in the following Examples). These sequences allow the Aβ variant to be secreted into the extracellular space by γ-secretase on the membrane. The sequence encoding SEQ ID NO: 13, for example, SEQ ID NO: 14 may be further included in the genetic construct according to the present invention, and may be located consecutive to the sequence encoding the variant.

The genetic constructs according to the present invention are intended for expression and/or administration in nerves. Specifically, the present invention aims for expression in cells expressed in the brain, including nerve tissue, more specifically, nerve cells, astrocytes, microglia, oligodendrocytes, and the like.

In the expression, the Aβ peptide variant is more preferably expressed in the extracellular space. This expression may prevent the extracellular accumulation of aggregated amyloid-β protein (Aβ), a major component of senile plaques.

In the present invention, the coding sequence encoding the Aβ peptide variant may comprise a sequence encoding γ secretase cleavage. An exemplary sequence of the γ secretase cleavage is shown in SEQ ID NO: 15, and a sequence encoding the sequence is shown in SEQ ID NO: 16. Through the sequence of γ secretase cleavage, the Aβ peptide variant may be expressed in the extracellular space, and thus, it is possible to achieve the therapeutic effect of diseases through the expression of the Aβ peptide variant in the extracellular space.

As used herein, the term "operably linked" refers to functional linkage between a nucleotide expression control sequence (such as a promoter, signal sequence, or array of transcription factor binding sites) and another nucleotide. The control sequence may control transcription and/or translation of the other nucleic acid sequence.

The genetic construct according to the present invention may express the Aβ peptide variant under the control of one or more promoters.

In the present invention, the promoter sequence may comprise a promoter sequence generally used in vector expression, a promoter sequence known specifically for neuron (referred to as neuron-specific promoter sequence), a promoter sequence for the purpose of overexpression, etc. For example, as common promoter sequences, cytomegalo virus (CMV) promoter, adenovirus late promoter, vaccinia virus 7.5K promoter, SV40 promoter, HSV tk promoter, RSV promoter, EF1 alpha promoter, metallothionein promoter, beta-actin promoter, human IL-2 gene promoter, human IFN gene promoter, human IL-4 gene promoter, human lymphotoxin gene promoter, human GM-CSF gene promoter, etc., may be used.

In particular, in the present invention, the promoter sequence may include a promoter sequence known specifically for neurons (referred to as neuron-specific promoter sequence). Examples of the neuron-specific promoter may include a human synapsin I (hSyn) promoter (for example, SEQ ID NO: 17), a mouse calcium/calmodulin-dependent protein kinase II (CaMKII) promoter (for example, SEQ ID NO: 18), a rat tubulin alpha I (Tuba1a) promoter (for example, SEQ ID NO: 19), a rat neuron-specific enolase (NSE) promoter (for example, SEQ ID NO: 20), and a human platelet-derived growth factor-beta chain (PDGF) promoter (for example, SEQ ID NO: 21), etc.

Genes regulated by neuron-specific promoters are expressed in most or all neurons but not in cells of other tissues. These nerve cells may include not only nerve cells but also cells included in nerve tissues such as the brain, such as astrocytes, microglia, oligodendrocytes, etc.

The overexpression promoter may include an EF-1α promoter (for example, SEQ ID NO: 22), a CAG promoter (for example, SEQ ID NO: 23), a CMV promoter (for example, SEQ ID NO: 24), etc.

Preferably, the promoter may be CAG, CaMKII, or human synapsin I (hSyn).

In addition, the genetic construct according to the present invention may contain one or more suitable transcriptional initiation, termination, enhancer sequences, efficient RNA processing signals, such as splicing and polyadenylation (polyA) signals that stabilize cytoplasmic mRNA, such as Kozak sequence; sequences that enhance translational efficiency or WPRE; sequences that enhance mRNA stability; and, if necessary, sequences that enhance secretion of the encoded product, etc.

For example, the genetic construct according to the present invention may also include an enhancer. The enhancer includes viral enhancers, including but not limited to CMV enhancers, WPRE enhancers, HPRE enhancers, CTE enhancers, or derivatives or hybrids thereof.

In addition, the genetic construct according to the present invention may include the Kozak sequence.

Packaging signals may be 5' inverted terminal repeats (ITRs) and 3' ITRs. For example, the genetic construct comprises AAV ITR sequences for use in AAV vectors. In an embodiment, the ITR is derived from a different AAV than the AAV supplying the capsid. In a preferred embodiment, the ITR sequence is derived from AAV2 or a deleted version thereof (ITR) which may be used for convenience and to accelerate regulatory approval. However, ITRs from other AAV sources may also be selected. When the source of the ITRs is from AAV2 and the AAV capsid is from another AAV source, the resulting vector may be termed a pseudotype. Typically, AAV vector genome comprises an AAV 5' ITR, any coding sequences and any regulatory sequences according to the present invention, and an AAV 3' ITR. However, other configurations of elements may also be suitable. A shortened version of the 5' ITR, which is termed ITR and in which the D-sequence and terminal resolution site (trs) are deleted, has been described. In another embodiment, full-length AAV 5' and 3' ITRs are used.

In some embodiments, the regulatory sequence comprises a polyadenylation (polyA) signal. In some embodiments, the polyA signal is a bovine growth hormone polyadenylation (bGH polyA) signal, a small polyA (SPA) signal, a human growth hormone polyadenylation (hGH polyA) signal, SV40 polyA signal, SV40 late polyA signal, or derivatives or hybrids thereof.

The genetic construct according to the present invention may further comprise a sequence encoding a signal peptide. The signal peptide may be any signal peptide that promotes proper folding or production, or may be a signal sequence that helps movement thereof to the cell membrane. In a preferred embodiment, the signal peptide may be any signal peptide disclosed herein. Specifically, the signal peptide may be any one selected from the group consisting of amyloid precursor protein (APP), human serum albumin, interleukin-2, CD5, immunoglobulin Kappa light chain, Gaussia Luciferase, trypsinogen, and prolactin.

It plays a role so that a sequence encoding an Aβ peptide variant having such a signal sequence is capable of being well expressed in the endoplasmic reticulum.

The genetic construct according to the present invention may be modified to the extent of maintaining the identity of the construct. That is, the term "identical" or percent "identity", in the context of two or more nucleic acids or polypeptide sequences, refers to two or more sequences or subsequences that are the same or have a specified percentage of amino acid residues or nucleotides that are the same (i.e., preferably 90%, 91%, 92%, 93%, 94%, 95%, 96%, 97%, 98%, 99% or higher identity) over a specific region (for example, any of the modified ORFs provided herein when compared and aligned for maximal correspondence over a comparison window or designated region) as measured using the BLAST and BLAST 2.0 sequence comparison algorithms using default parameters as described below or by manual alignment and visual inspection (see, for example, the NCBI website, etc.). In other words, modification of the genetic construct within the range of maintaining the functional effect of the desired Aβ peptide variant is included within the scope of the present invention.

The present invention provides a recombinant expression vector comprising the genetic construct. The genetic construct and expression vector described herein may be used for the treatment and amelioration of neurodegenerative diseases.

The recombinant expression vector refers to a plasmid, viral vector or other vehicle known in the art into which a nucleic acid encoding a genetic construct is capable of being inserted and the nucleic acid is capable of being expressed in a host cell. Preferably, the recombinant expression vector may be a viral vector. Examples of the viral vector include, but are not limited to, an adenovirus vector, an adeno-associated virus (AAV) vector, a herpes virus vector, an avipoxvirus vector, a lentivirus vector, and the like. In particular, methods using lentivirus or adeno-associated virus (AAV) are preferred.

Adeno-associated virus (AAV) viral vectors are AAV DNase-resistant particles having an AAV protein capsid within which nucleic acid sequences are packaged for delivery to target cells. The AAV capsid is composed of 60 capsid (cap) protein subunits, VP1, VP2 and VP3, that are arranged in icosahedral symmetry in an approximate ratio of 1 : 1 : 10 to 1 : 1 : 20 depending on the selected AAV. The AAV capsid may be selected from those known in the art, including variants.

The vector "rAAV" refers to a viral particle composed of at least one AAV capsid protein and an encapsidated polynucleotide rAAV vector comprising a heterologous polynucleotide (i.e., a polynucleotide that is not a wild-type AAV genome, such as a transgene to be delivered to a mammalian cell). The rAAV particles may have any AAV serotype, including any modification, derivative or pseudotype (for example, AAV1, AAV2, AAV3, AAV4, AAV5, AAV6, AAV7, AAV8, AAV9 or AAV10 or derivatives/modifications/pseudotypes thereof). The AAV serotypes and derivatives/variants/pseudotypes and methods of producing these serotypes/derivatives/variants/pseudotypes are known in the art (see, e.g., Asokan et al., Mol. Ther. 20(4) :699-708 (2012)]). In some embodiments, the rAAV particles comprise a capsid protein from an AAV capsid serotype selected from AAV1, AAV2, AAV3, AAV4, AAV5, AAV6, AAV7, AAV8, AAV9, AAV10, AAV11, AAV12, AAV13, AAV14, AAV15, AAV16, AAV.rh8, AAV.rh10, AAV.rh20, AAV.rh39, AAV.Rh74, AAV.RHM4-1, AAV.hu37, AAV.Anc80, AAV.Anc80L65, AAV.7m8, AAV.PHP.B, AAV.PHP.eB, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, and AAV.HSC16. In some embodiments, the rAAV particles comprise a capsid protein that is a derivative, modification or pseudotype of AAV1, AAV2, AAV3, AAV4, AAV5, AAV6, AAV7, AAV8, AAV9, AAV10, AAV11, AAV12, AAV13, AAV14, AAV15, AAV16, AAV.rh8, AAV.rh10, AAV.rh20, AAV.rh39, AAV.Rh74, AAV.RHM4-1, AAV.hu37, AAV.Anc80, AAV.Anc80L65, AAV.7m8, AAV.PHP.B, AAV.PHP.eB, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16 capsid protein.

The rAAV particles of the present disclosure may have any serotype or any combination of serotypes (for example, a population of rAAV particles comprising two or more serotypes, for example, comprising two or more of rAAV2, rAAV8 and rAAV9 particles). In some embodiments, the rAAV particle is rAAV1, rAAV2, rAAV3, rAAV4, rAAV5, rAAV6, rAAV7, rAAV8, rAAV9, rAAV10 or another rAAV particle or a combination of two or more thereof. In some embodiments, the rAAV particle is a rAAV8 or rAAV9 particle. In some embodiments, the rAAV particles comprise capsid proteins from two or more serotypes selected from AAV1, AAV2, AAV3, AAV4, AAV5, AAV6, AAV7, AAV8, AAV9, AAV10, AAV11, AAV12, AAV13, AAV14, AAV15 and AAV16, AAV.rh8, AAV.rh10, AAV.rh20, AAV.rh39, AAV.Rh74, AAV.RHM4-1, AAV.hu37, AAV.Anc80, AAV.Anc80L65, AAV.7m8, AAV.PHP.B, AAV.PHP.eB, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, and AAV.HSC16. In some embodiments, the rAAV particles comprise capsid proteins that are derivatives, mutations or pseudotypes of two or more serotypes selected from AAV1, AAV2, AAV3, AAV4, AAV5, AAV6, AAV7, AAV8, AAV9, AAV10, AAV11, AAV12, AAV13, AAV14, AAV15, and AAV16, AAV.rh8, AAV.rh10, AAV.rh20, AAV.rh39, AAV.Rh74, AAV.RHM4-1, AAV.hu37, AAV.Anc80, AAV.Anc80L65, AAV.7m8, AAV.PHP.B, AAV.PHP.eB, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, and AAV.HSC16 capsid proteins.

In some embodiments, the rAAV particle has an AAV capsid protein of a serotype selected from the group consisting of AAV1, AAV2, AAV3, AAV4, AAV5, AAV6, AAV7, AAV8, AAV9, AAV10, AAV11, AAV12, AAV13, AAV14, AAV15, and AAV16, or a derivative, mutation, or pseudotype thereof. In some embodiments, the rAAV particle has an AAV capsid protein of a serotype of AAV8, AAV9, or a derivative, mutation, or pseudotype thereof. In some embodiments, the rAAV particle has an AAV capsid protein of a serotype selected from the group consisting of AAV7, AAV8, AAV9, AAV.rh8, AAV.rh10, AAV.rh20, AAV.rh39, AAV.Rh74, AAV.RHM4-1, AAV.hu37, AAV.PHP.B, AAV.PHP.eB, and AAV.7m8.

Preferably, the rAAV is any one selected from the group consisting of AAV2, AAV7, AAV8, AAV9, AAV.rh8, AAV.rh10, AAV.rh20, AAV.rh39, AAV.Rh74, AAV.RHM4-1, AAV.hu37, AAV.PHP.B, AAV.PHP.eB, and AAV.7m8.

According to an embodiment, the AAV vector in regards to the present invention is able to cross the blood-brain-barrier (BBB). AAV according to the present invention may be used to efficiently and widely transduce neurons in the CNS.

The adeno-associated virus (AAV) is suitable for the gene delivery system of the present invention because it has the ability to transduce non-dividing cells and transduce various types of cells. Detailed descriptions of the production and the use of AAV vectors are disclosed in detail in U.S. Patent Nos. 5,139,941 and 4,797,368.

Advantageously, recombinant AAV2 evokes a minimal immune response in host organisms and mediates long-term transgene expression capable of persisting for at least one year after vector administration.

As used herein, the term "recombinant AAV (rAAV) vector" refers to a recombinant AAV-derived nucleic acid containing at least one terminal repeat sequence.

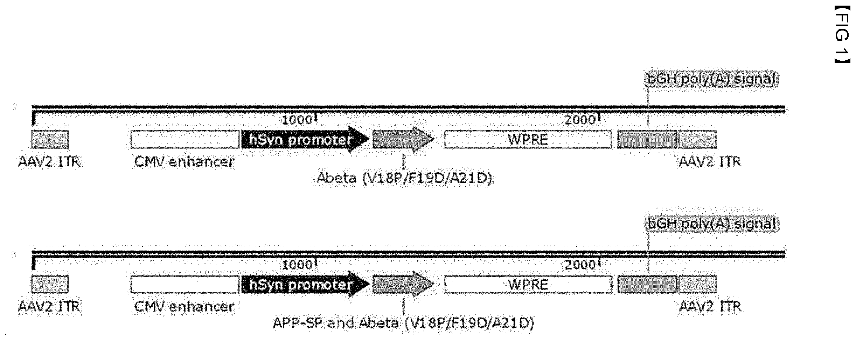

Preferred embodiments of vectors containing the above gene construct are shown in FIG. 1.

Specifically, according to FIG. 1, an expression vector may be constructed to comprise the hSyn promoter processed in the CMV enhancer and an Aβ42 variant having, for example, V18P/F19D/A21D mutation.

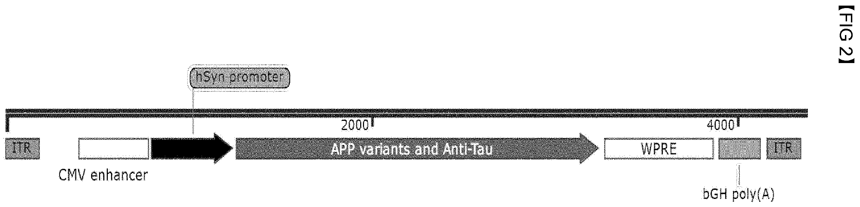

A preferred embodiment of a vector comprising the above gene construct is shown in FIG. 2. Specifically, according to FIG. 2, an expression vector may be constructed to comprise the hSyn promoter processed in the CMV enhancer and an Aβ variant having the above-described mutation.

The present invention provides a peptide comprising at least one mutation selected from the group consisting of V689P, F690D, and A692D based on amyloid precursor protein.

The present invention also provides a polynucleotide encoding the peptide.

The present invention provides a peptide comprising any one or more mutations selected from the group consisting of V18P, F19D, and A21D based on Aβ42.

The present invention also provides a polynucleotide encoding the peptide.

The peptide sequences of the Aβ variants, specifically variants of amyloid precursor protein, and the Aβ42 variants, and the polynucleotide sequences encoding the peptide sequences according to the present invention are as described above.

The peptide sequence exhibits excellent preventive, ameliorative and therapeutic effects on neurodegenerative diseases by slowing down or hindering wt Aβ polymerization and reducing toxicity.

The present invention provides a pharmaceutical composition comprising the genetic construct or the recombinant expression vector, and a pharmaceutically acceptable carrier.

The present invention provides a pharmaceutical composition for preventing or treating neurodegenerative diseases comprising the genetic construct and/or the recombinant expression vector.

In the present invention, the neurodegenerative disease is any one or more selected from the group consisting of Alexander disease, Alpers disease, Alzheimer's disease, amyotrophic lateral sclerosis (ALS), ataxia-telangiectasia, neuronal ceroid lipofuscinoses, Batten disease, bovine spongiform encephalopathy (BSE), Canavan disease, cerebral palsy, Cockayne syndrome, corticobasal degeneration, Creutzfeldt-Jakob disease, frontotemporal lobe degeneration, Gaucher disease, Huntington's disease, HIV-associated dementia, Kennedy disease, Krabbe disease, Lewy body dementia, lysosomal storage disorder, neuroborreliosis, Machado-Joseph disease, motor neuron disease, multisystem atrophy, multiple sclerosis, multiple sulfatase deficiency, mucolipidosis, narcolepsy, Niemann-Pick type C, Niemann-Pick disease, Parkinson's disease, Pelizaeus-Merzbacher disease, Pick's disease, Pompe disease, primary lateral sclerosis, prion disease, progressive supranuclear palsy, Refsum disease, Sandhoff disease, Schilder disease, subacute combined degeneration of the spinal cord secondary to pernicious anemia, Spielmeyer-Vogt-Sjogren-Batten disease, spinocerebellar ataxia, spinal muscular atrophy, Steele Richardson Olszewski syndrome, spinal cord syphilis, and Tay-Sachs disease.

In a preferred embodiment, the neurodegenerative disease is any one or more selected from the group consisting of Alzheimer's disease, Huntington's disease, Parkinson's disease and motor neuron disease. More preferably, the neurodegenerative disease is Alzheimer's disease.

Accordingly, the present invention provides a method for treating, preventing, or alleviating neurodegenerative diseases in a subject, or for promoting nerve regeneration and/or survival in a subject. The method comprises administering a therapeutically effective amount of the genetic construct or the recombinant expression vector, to a subject in need of such treatment.

It will be appreciated that the genetic construct or the recombinant expression vector is capable of being used in the medicament, which is usable as a monotherapy to treat, alleviate or prevent neurodegenerative diseases, or to promote nerve regeneration and/or survival. Alternatively, the genetic construct or recombinant expression vector according to the present invention may be used in addition to or in combination with known therapies to treat, alleviate, or prevent neurodegenerative diseases, or to promote nerve regeneration and/or survival.

Further, the present invention provides a genetic construct or a recombinant expression vector for use in the treatment of neurodegenerative diseases.

In addition, the present invention provides the use of the genetic construct or the recombinant expression vector in the manufacture of a medicament for use in the treatment of neurodegenerative diseases.

The genetic construct or recombinant expression vector according to the present invention may be combined in a composition having several different forms, in particular depending on the manner in which the composition is to be used. Thus, for example, the composition may be in the form of a powder, tablet, capsule, liquid, ointment, cream, gel, hydrogel, aerosol, spray, micellar solution, transdermal patch, liposomal suspension, or any other suitable form capable of being administered to a human or animal in need of treatment. It will be appreciated that the carrier of a medicament according to the present invention should be well familiarly used by the subject to whom/which it is given.

In a preferred embodiment, a medicament according to the present invention may be administered to a subject by injection into the blood stream, nerves or directly into the site in need of treatment. For example, the medicament is configured to cross the blood-brain-barrier. Injections may be intravenous (bolus or infusion) or subcutaneous (bolus or injection) or intradermal (bolus or injection).

It will be appreciated that the amount of genetic construct or recombinant expression vector required is determined by its biological activity and bioavailability, which in turn depends on the mode of administration, the physicochemical properties of the genetic construct or recombinant expression vector, and whether the genetic construct or recombinant expression vector is being used as a monotherapy or in combination therapy. The frequency of administration will also be affected by the half-life of the circulating polypeptide in the subject being treated. The optimal dosage to be administered may be determined by a person skilled in the art, and will vary with the particular genetic construct or recombinant expression vector being used, the strength of the pharmaceutical composition, the mode of administration, and the progression or stage of the disorder. Depending on the particular subject being treated, additional factors including the subject's age, weight, sex, diet, and time of administration may require adjusting the dosage.

In general, depending on the genetic construct or recombinant expression vector used, a daily dose of 0.001 µg/kg body weight to 10 mg/kg body weight, or 0.01 µg/kg body weight to 1 mg/kg body weight of the construct or vector according to the present invention may be used to treat, alleviate or prevent neurodegenerative diseases.

The genetic construct or recombinant expression vector may be administered before, during, or after the onset of the disease and/or disorder.

Known procedures, such as those commonly employed in the pharmaceutical industry (for example, in vivo experiments, clinical trials, and the like) may be used to determine specific formulations and precise therapeutic regimens (for example, daily dose and frequency of administration of the formulation) of the genetic constructs or recombinant expression vectors according to the present invention.

In the present invention, "subject" may be a vertebrate, mammal, or domestic animal. Therefore, the compositions and medicaments according to the present invention may be used to treat any mammal, such as livestock (for example, horses), pets, or may be used in other veterinary applications. Most preferably, however, the subject is a human.

A "therapeutically effective amount" of the genetic construct, recombinant expression vector, or pharmaceutical composition, when administered to a subject, is any amount that is above-described amount necessary to treat neurodegenerative diseases, or to produce a desired effect, such as promoting nerve regeneration and/or survival.

For example, a therapeutically effective amount of the genetic construct, recombinant expression vector or pharmaceutical composition to be used may be from about 0.01 mg to about 800 mg, and preferably from about 0.01 mg to about 500 mg. Preferably, the amount of the genetic construct, recombinant expression vector or pharmaceutical composition is from about 0.1 mg to about 250 mg.

As referred to herein, the term "pharmaceutically acceptable carrier" is any known compound or a combination of known compounds known to those skilled in the art that is useful in the formulation of pharmaceutical composition.

The pharmaceutical carrier may be liquid, and the pharmaceutical composition is in the form of a solution. A liquid carrier is used to prepare solutions, suspensions, emulsions, syrups, elixirs and pressurized compositions. A genetic construct or recombinant expression vector according to the present invention may be dissolved or suspended in a pharmaceutically acceptable liquid carrier such as water, an organic solvent, a mixture of both, or a pharmaceutically acceptable oil or fat. The liquid carrier may contain other suitable pharmaceutical additives such as solubilizers, emulsifiers, buffers, preservatives, sweeteners, flavoring agents, suspending agents, thickening agents, colorant, viscosity modifiers, stabilizers or osmotic pressure-regulating agents. Suitable examples of liquid carriers for oral and parenteral administration include water (partially containing additives as above such as cellulose derivatives, preferably sodium carboxymethyl cellulose solution), alcohols (including monohydric and polyhydric alcohols such as glycols) and derivatives thereof, and oils (for example, fractionated coconut oil and arachis oil). For parenteral administration, the carrier may also be an oily ester such as ethyl oleate and isopropyl myristate. Sterile liquid carriers are useful in sterile liquid form compositions for parenteral administration. The liquid carrier for pressurized compositions may be halogenated hydrocarbon or other pharmaceutically acceptable propellent.

Liquid pharmaceutical compositions which are sterile solutions or suspensions may be utilized by, for example, brainstem, intramuscular, intradural, epidural, intrathecal, intraperitoneal, intravenous and subcutaneous injection. The gene construct or recombinant expression vector may be prepared as sterile solid compositions capable of being dissolved or suspended by using sterile water, saline, or other suitable sterile injectable media for administration.

The genetic construct, recombinant expression vector, and pharmaceutical composition of the present invention may be administered orally in the form of sterile solutions or suspensions containing other solutes or suspending agents (for example, sufficient saline or glucose to render the solution isotonic), bile salts, acacia, gelatin, sorbitan monooleate, polysorbate 80 (oleate ester of sorbitol and anhydride thereof copolymerized with ethylene oxide), and the like. The gene construct, recombinant expression vector or pharmaceutical composition according to the present invention may also be administered orally in the form of liquid or solid compositions. Compositions suitable for oral administration include solid forms such as pills, capsules, granules, tablets, and powders, and liquid forms such as solutions, syrups, elixirs, and suspensions. Forms useful for parenteral administration include sterile solutions, emulsions, and suspensions.

All features recited herein (including all appended claims, abstracts and figures) and/or all steps of any method or process so disclosed may be combined with any of the above aspects in any combination, except for combinations in which at least some of these features and/or steps are mutually exclusive.

### [Advantageous Effects]

The genetic construct of the present invention exhibits excellent preventive, ameliorative and therapeutic effects on neurodegenerative diseases by secreting Aβ variants to slow down or interfere with wt Aβ polymerization in the human body and reduce toxicity.

### [Description of Drawings]

In order to provide a better understanding of the present invention, and to show how embodiments thereof may be practiced, reference will now be made to the accompanying drawings by way of example:
FIG. 1 shows an example of an AAV vector comprising nucleotide sequences of an Aβ42 peptide variant. In an exemplary embodiment, the genetic construct comprises the hSyn promoter and nucleotide sequences of the Aβ42 peptide variant.
FIG. 2 shows an example of an AAV vector comprising nucleotide sequences of the Aβ peptide variant. In an exemplary embodiment, the genetic construct comprises the hSyn promoter and the nucleotide sequences of the Aβ peptide variant.
FIG. 3 shows the formation of aggregates in the ThT assay
FIG. 4 shows the competitive aggregate formation.
FIG. 5 shows the results of confirming disassembly of fibrils of Aβ42 (V18P/F19D/A21D) variants.
FIG. 6 shows the results of confirming the cytotoxicity of the Aβ42 (V18P/F19D/A21D) variants.
FIG. 7 shows the results of immunoprecipitation to measure the secretion of Aβ in the media after pAAV-hSyn-V18P/A21D or pAAV-hSyn-V18P/F19D/A21D was transfected into N2a cells.
FIG. 8 shows shows the results of confirming colocalization through co-staining for Aβ42 (V18P/F19D/A21D, (6E10, green)) variants and endogenous mouse APP (Y188, red) by administering AAV containing the peptide sequence according to the present invention.
FIG. 9 shows the results of confirming changes in plaque burden by administering AAV containing the peptide sequence according to the present invention.
FIG. 10 shows the results of confirming changes in microglia and astrocytes by administering AAV containing the peptide sequence according to the present invention.
FIG. 11 shows the results of quantifying changes in microglia and astrocytes by administering AAV containing the peptide sequence according to the present invention.
FIG. 12 shows the results of confirming the therapeutic effect by administering AAV containing the peptide sequence according to the present invention in the Morris Water Maze test.

### [Best Mode]

Hereinafter, the present disclosure will be described in more detail through Examples. However, these Examples are provided to illustrate the present disclosure by way of example, and the scope of the present disclosure is not limited to these Examples.

### Example 1. Design of AAV vector comprising Aβ42 variant

An AAV vector comprising nucleotide sequences of an Aβ42 peptide variant was constructed, and a schematic diagram thereof is shown in FIG. 1.

The Kozak sequence and *Gaussia luciferase* signal peptide (GLSP) DNA were bound to the N-terminus of Aβ-CTF (C-terminal fragment) and cut by using restriction enzymes HindIII and EcoRV, followed by cloning into pAAV vector containing the *CAG* promoter and woodchuck poliovirus response element (WPRE) sequences to prepare pAAV-GLSP-Aβ-CTF. Then, the CMV enhancer was amplified, then cut by using restriction enzymes XhoI and ApaI, and attached to the pAAV-hSyn-eGFP vector to prepare pAAV-CMV enhancer-hSyn-eGFP. Then, the CMV enhancer and hSyn promoter were amplified from pAAV-CMV enhancer-hSyn-eGFP, cut by using restriction enzymes KpnI and HindIII, followed by cloning into a pAAV vector containing WPRE and poly-A sequences to prepare a pAAV-CMV enhancer-hSyn-WPRE-polyA vector. Finally, DNA expressing Aβ (V18P/A21D) (SEQ ID NO: 11) or Aβ (V18P/F19D/A21D) (SEQ ID NO: 12), which is a variant of Aβ, was amplified from pAAV-GLSP-Aβ-CTF, and cut by using restriction enzyme HindIII and EcoRV, followed by cloning into pAAV-CMV enhancer-hSyn-WPRE-polyA to create pAAV-hSyn-GLSP-Aβ(V18P/A21D)-KK-WPRE-polyA or pAAV-hSyn-GLSP-Aβ(V18P/F19D/A21D)-KK-WPRE-polyA, respectively.

Similar to the production process in the above step, as shown in Table 1, AAV vectors containing nucleotide sequences of Aβ42 peptide variants and AAV vectors containing nucleotide sequences of Aβ (APP) peptide variants and tau inhibitor peptides were further designed (using AAV-PhP.eB) .

**[Table 1]**

| **Promoter Sequence** | **Nucleotide sequence of Aβ42 peptide variant** |
|---|---|
| CaMKII promoter | V18P/A21D mutation(SEQ ID NO: 9) |
| CaMKII promoter | V18P/F19D/A21D mutation(SEQ ID NO: 10) |
| hSyn promoter | V689P/A692D mutation(SEQ ID NO: 3) |
| hSyn promoter | V689P/F690D/A692D mutation(SEQ ID NO: 4) |
| CaMKII promoter | V689P/A692D mutation(SEQ ID NO: 3) |
| CaMKII promoter | V689P/F690D/A692D mutation (SEQ ID NO: 4) |

### Example 2. In silico analysis

The AGGRESCAN program was used to analyze hot spots (aggregation-propensity values per each residue) in which aggregation of polypeptides may occur due to mutations.

Specifically, AGGRESCAN is based on an aggregation-propensity scale for natural amino acids derived from in vivo experiments and on the assumption that short and specific sequences modulate protein aggregation. The algorithm is shown to identify a series of protein fragments involved in the aggregation of disease-related proteins and to predict the effect of genetic mutations on their deposition propensities. It also provides new insights into the differential aggregation properties displayed by globular proteins, natively unfolded polypeptides, amyloidogenic proteins and proteins found in bacterial inclusion bodies.

It was confirmed through the analysis using the above program that the V18P/A21D mutation, and V18P/F19D/A21D mutation in the Aβ42 peptide sequences correspond to mutation factors capable of significantly reducing the aggregation level when compared to WT. That is, the V18P/A21D mutation and the V18P/F19D/A21D mutation showed a very good effect on aggregation inhibition. Specifically, it was confirmed that an excellent effect was exhibited when in the V18P/A21D mutation and V18P/F19D/A21D, valine (V) and phenylalanine (F) were replaced with proline (P) and aspartic acid (D) (well-known β-sheet blocking amino acids) to reduce the peptide's propensity to adopt the β-sheet conformation, and alanine was also replaced with aspartic (D) acid (charged residue) to increase solubility.

### Example 3. Confirmation of epidemiology of Aβ autoaggregation and competition with WT peptide

### (1) Preparation of WT Aβ42 or variant peptide stocks

Synthetic Aβ42 WT or variant peptides were purchased from Biomatik. To prepare stock solutions of aggregate-free Aβ42 WT or variant peptides, lyophilized Aβ42 peptides (WT, V18P/F19D/A21D mutation) were dissolved in hexafluoroisopropanol (HFIP). The Aβ-HFIP solution was incubated at room temperature (RT) for 30 minutes and then divided into aliquots. HFIP was evaporated overnight in a fume hood and then transferred to SpeedVac for 1 hour to remove any remaining traces of HFIP. Tubes containing the peptide film were kept over desiccant at -20°C until used. Immediately prior to experimental use, lyophilized peptides were dissolved in 0.1% NH₄OH to a final concentration of 100 µM and sonicated for 10 minutes in a bath sonicator.

### (2) ThT assay to test epidemiology of Aβ autoaggregation and competition with WT peptide

Autoaggregation of Aβ42 WT or variant peptides was tested at a starting concentration of 10 µM in PBS containing 50µM Thioflavin T (ThT). ThT fluorescence was measured using a Biotek Synergy H1 reader at an excitation wavelength of 440 nm and emission wavelength of 485 nm. Reactions were incubated at 37°C and then shaken for 5 seconds every 10 minutes prior to reading the fluorescence. Competition between variant and WT Aβ was performed similarly but using a starting concentration of 10 µM for each peptide in the mixture (1 : 1, WT : variant), and error bars show the standard deviation of repeated measurements.

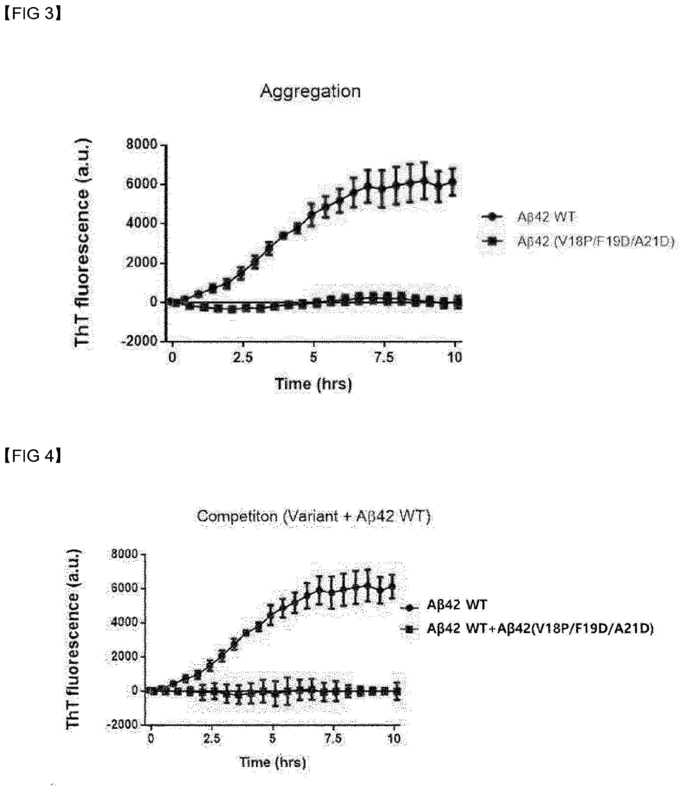

Results thereof are shown in FIGS. 3 and 4.

FIG. 3 shows the formation of aggregates in the ThT assay, and FIG. 4 shows the competitive aggregate formation, and these results showed that the Aβ42 peptide variants reduced fibrillization of Aβ42 WT. Specifically, as could be seen in FIG. 3, Thioflavine-T (ThT) assay for autoaggregation of Aβ42 peptide variants compared to Aβ42 WT showed no autoaggregation in any of the three variants during the reaction. Also, for comparison, it was shown that Aβ42 WT reached the peak of ThT binding within 7 hours and generated aggregation.

As could be seen in FIG. 4, in the competition assay to test the inhibition of Aβ42 WT aggregation, each Aβ42 peptide variant was mixed with Aβ42 WT at a ratio of 1 : 1 and cultured with ThT, Aβ42 (V18P/F19D/A21D) completely attenuated the aggregation of the Aβ42 WT peptide.

From the above results, it was confirmed that the Aβ peptide variants according to the present invention could interfere with or inhibit wt Aβ and may exhibit excellent disease improvement and therapeutic effects by the disassembly of the existing aggregated Aβ plaque.

### Example 4. Confirmation of disassembly of fibrils of Aβ42 (V18P/F19D/A21D) variants

Aβ42 WT was exposed to monomeric V18P/F19D/A21D peptides at various concentrations, and concentration-dependent changes in ThT fluorescence were confirmed. Specifically, autoaggregation of Aβ42 WT and/or variant peptides was tested by treating 10 µM of Aβ42 WT with different concentrations of 5 µM, 10 µM and 20 µM of variant peptides in PBS containing 5 µM Thioflavin T (ThT) (mixed in a ratio of 1 : 1). ThT fluorescence was measured using a Biotek Synergy H1 reader at an excitation wavelength of 440 nm and emission wavelength of 485 nm. Reactions were incubated at 37°C and fluorescence was measured without shaking every 10 hours during 40 hours of incubation at 37°C. Error bars show the standard deviation of repeated measurements.

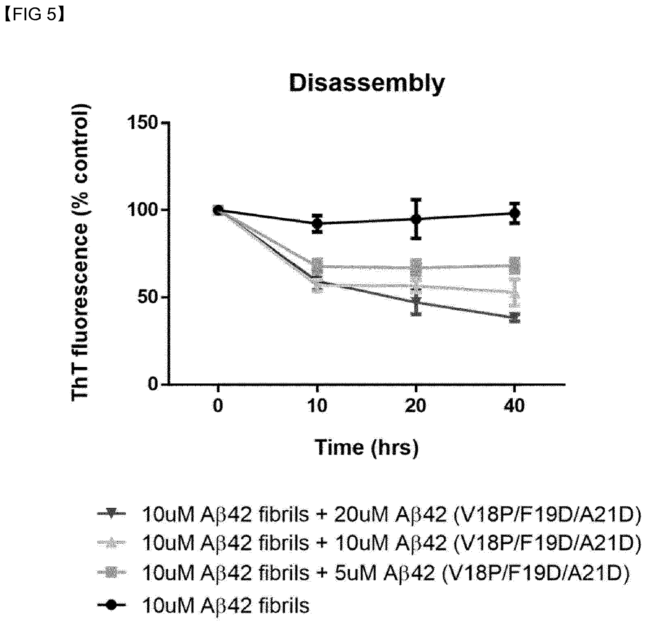

### Experimental results thereof are shown in FIG. 5.

As could be confirmed from FIG. 5, as compared to WT Aβ42 fibrils alone, incubation with the V18P/F19D/A21D peptide produced a concentration-dependent reduction in ThT fluorescence.

These results indicate that the Aβ42 variant, according to the present invention, is able to disassemble WT Aβ42 fibrils to remove previously formed Aβ plaques when the Aβ42 variants are expressed in the brain.

### Example 5. Confirmation of cytotoxicity of Aβ42 variants

Through the MTS assay, the cytotoxicity of Aβ42 was confirmed.

Specifically, for MTS analysis, 1) Aβ42 WT, 2) V18P/A21D mutation sequence, 3) V18P/F19D/A21D mutation sequence, 4) Aβ42 WT + V18P/A21D mutation sequence, and 5) Aβ42 WT + V18P/F19D/A21D mutation sequences were incubated at 100 µM each at 4°C for 24 hours to form oligomers.

N2a cells were seeded into a 96-well plate, and then 10 µL of each formulation's oligomer (WT Aβ alone, variant alone or WT Aβ + variant) was added to 90 µL of the culture medium to a final concentration of 10 µM. Cell viability was determined 24 hours after treatment using a MTS assay. Specifically, assays were performed by adding 20 µl of AQueous One Solution Reagent directly to culture wells, incubating for 2 hours at 37°C in 5% CO₂ atmosphere and then recording absorbance at 490 nm using a spectrophotometer.

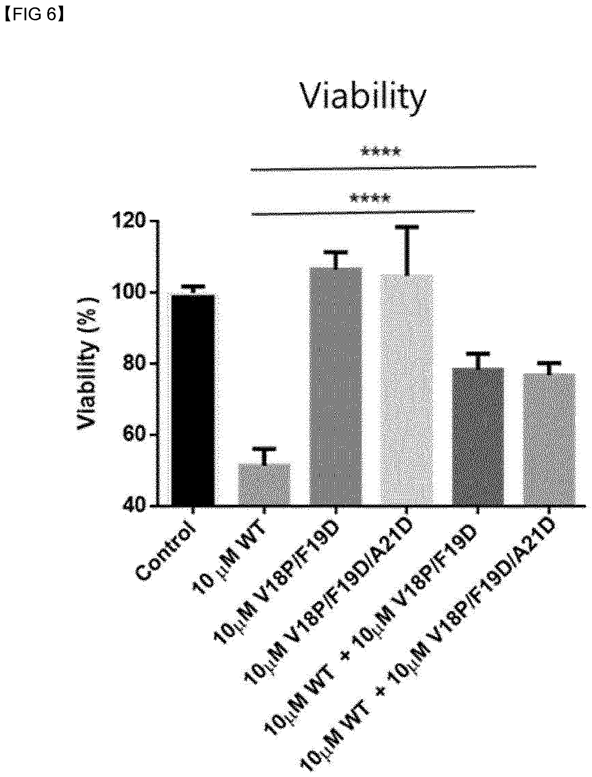

Results thereof are shown in FIG. 6.

As could be confirmed through FIG. 6, unlike Aβ42 WT, it was confirmed that neither V18P/F19D/A21D nor V18P/A21D variants caused toxicity of N2a cells by themselves, which corresponded to a level similar to that of the control group, confirming that there was no cytotoxicity.

In contrast, co-incubation of WT Aβ42 with variants (V18P/F19D/A21D or V18P/A21D) during oligomeric Aβ42 formation showed better cell viability than Aβ42 WT (ANOVA, *p < 0.05, **p < 0.01., ***p < 0.001, ****p < 0.0001. All data are presented as mean ± SEM).

### Example 6. Confirmation of Aβ immunoprecipitation in conditioned media

Transformation was performed by using pAAV-hSyn-GLSP-Aβ(V18P/A21D)-KK-WPRE-polyA, or pAAV-hSyn-GLSP-Aβ(V18P/F19D/A21D)-KK-WPRE-polyA vector prepared in Example 1 above. Then, the conditioned media from the transfected N2a cells (6 mL) were incubated with the Dynabead-6E10 complex in a rotator overnight at 4°C. The following day, beads were collected using magnetic separation to remove the supernatant, and the beads were washed 3 times with PBS containing 0.02% Tween-20. Immunoprecipitated complexes were eluted with 50 mM glycine (pH 2.8), and samples were then confirmed using the 6E10 antibody that reacts to human Aβ.

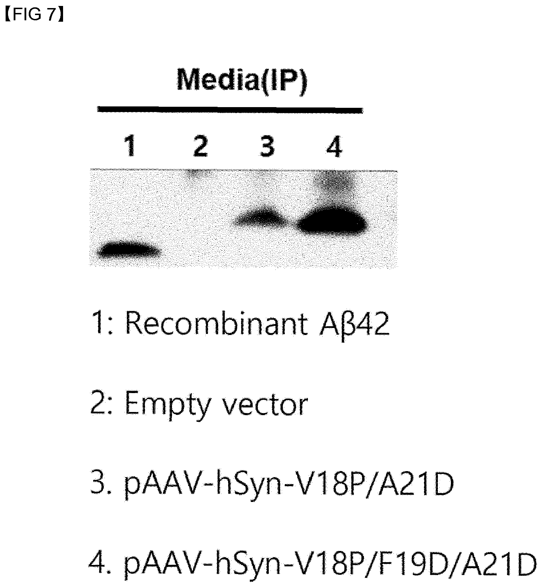

Results thereof are shown in FIG. 7.

As could be seen in FIG. 7, the administration of the variant according to the present invention clearly showed the measurement results regarding the secretion of Aβ in the immunoprecipitation results.

These results show that AAV secretes variants suitably to exhibit therapeutic effects on Aβ aggregation or Aβ plaque formation that occurs outside of neurons.

### Example 7. Confirmation of therapeutic effect according to administration of AAV vector comprising Aβ42 variant sequences

### (1) Animal model

The AAV vectors prepared in Example 1 (pAAV-CMV enhancer-hSyn-GLSP-Aβ(V18P/F19D/A21D)-KK-WPRE-polyA) and the control AAV (pAAV-CMV enhancer-hSyn-WPRE-polyA) were respectively injected into the brain or vein of 3xTg AD mice, and AD-related markers were observed. In 9 months or 12 months after virus injection, 3xTg AD mice were subjected to behavioral tests and were sacrificed at the end of the tests, and their brains were processed for further biochemical and immunohistochemical analysis.

### (2) Aβ biochemical and immunohistochemical analysis

As for the plaque burden, Aβ-40 and -42 were extracted from the frontal cortex using guanidine, which is used for brain deposit extraction, and quantitative Aβ40 and -42 accumulation was confirmed by Aβ ELISA. Amyloid plaques elicit a pronounced neuroimmune response in which hypertrophic astrocytes and microglia migrate or divide to surround cored deposits. Glial fibrillary acidic protein (GFAP) immunostaining was used to detect astrocytes and Ibal to detect microglia.

### (3) Confirmation of colocalization and confirmation of changes in microglia and astrocyte through co-staining for Aβ42 (V18P/F19D/A21D) variants and endogenous mouse APP (Y188, red)

Brain slices obtained after sacrificing 3xTg AD mice after 12 months were rinsed with tris-buffered saline (TBS) and blocked with TBS containing 0.1% Triton X-100 (TBS-T), followed by overnight incubation at 4°C with GFAP antibody, Ibal antibody, or human Aβ antibody-6E10 and mouse Aβ antibody-Y188. Then, sections were incubated with Alexa Fluor-568 Gt anti-Rb and Alexa Fluor-488 Gt anti-mouse secondary antibodies and confirmed by fluorescence microscopy.

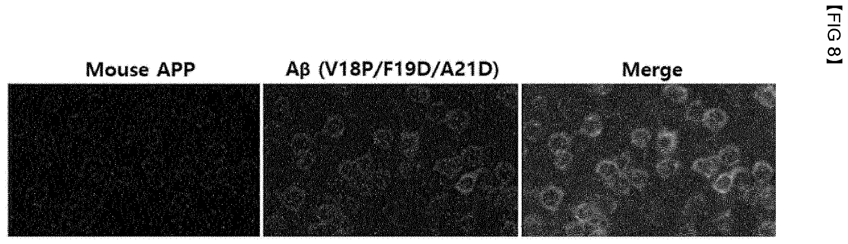

Co-staining for variant Aβ (V18P/F19D/A21D; 6E10, green) delivered by pAAV-hSyn-GLSP-Aβ(V18P/F19D/A21D)-KK-WPRE-polyA and endogenous mouse APP (Y188, red) showed confirmation of membrane transport of variant peptide in cortical neurons, which is shown in FIG. 8.

As could be confirmed in FIG. 8, it was confirmed that the delivered variant Aβ (V18P/F19D/A21D; 6E10, green) and the endogenous mouse APP (Y188, red) showed colocalization, through which the membrane transport of the peptide in cortical neurons was confirmed.

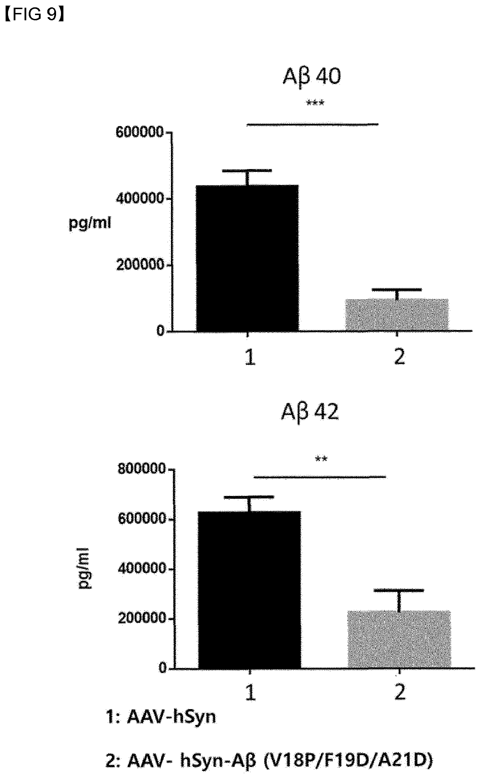

To confirm changes in plaque burden, mice were transfected with pAAV-CMV enhancer-hSyn-GLSP-Aβ(V18P/F19D/A21D)-KK-WPRE-polyA and sacrificed 12 months after the transfection, and changes in the amounts of Aβ 40 and Aβ 42, which are major components of plaque, were confirmed by ELISA. As shown in FIG. 9, AAV treatment according to the present invention showed an effect of improving plaque burden by reducing about 78% of Aβ40 and about 64% of Aβ42 (t-test, *p < 0.05, **p < 0.01, ***p < 0.001, ****p < 0.0001. All data are presented as mean ± SEM).

To confirm changes in microglia and astrocytes, mice were transfected with pAAV-hSyn-GLSP-Aβ(V18P/F19D/A21D)-KK-WPRE-polyA and sacrificed 12 months after the transfection, and changes in microglia and astrocytes were confirmed.

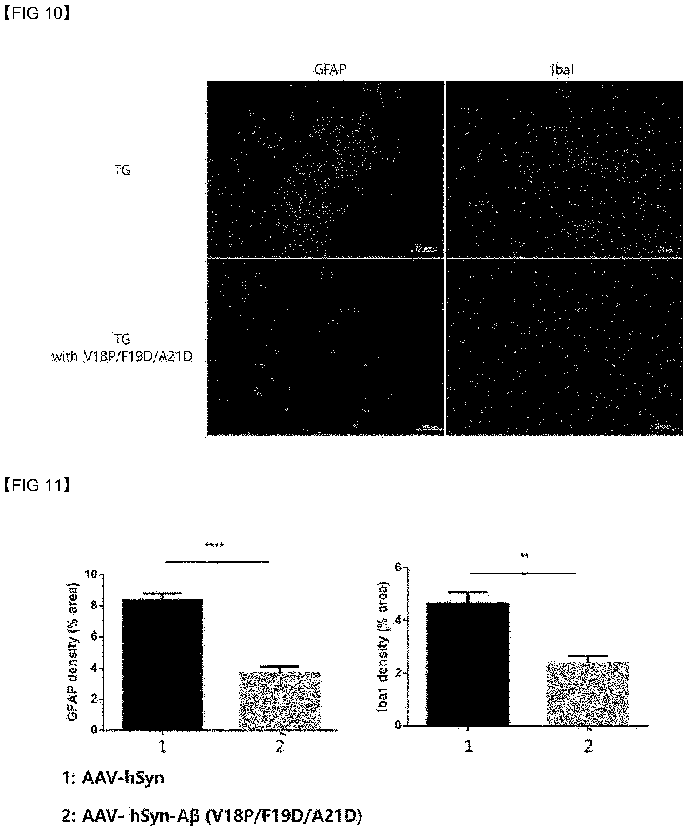

Specifically, the results of confirming changes in microglia and astrocytes through immunostaining are shown in FIG. 10.

GFAP immunostaining (red) was used to detect astrocytes, and Ibal (red) was used to detect microglia. Amyloid plaques elicit a remarkable neuroimmune response, and depending on the response, hypertrophic astrocytes and microglia migrate or differentiate into surround cored deposits. Under most conditions, the extent of glial induction parallels the severity of amyloid load. As could be confirmed from the figures, AAV treatment according to the present invention showed an improvement effect on microglia and astrocytes.

The numerical result values of the staining test are shown in FIG. 11.

As could be confirmed in FIG. 11, the results of quantification of the area occupied by GFAP and IBA1 staining showed that both the size of glial foci and the number of surrounding cells were reduced by the treatment of AAV according to the present invention (t-test, *p < 0.05, **p < 0.01, ***p < 0.001, ****p < 0.0001. All data are presented as mean ± SEM).

### (4) Confirmation of spatial perception ability and memory improvement effect through Morris Water Maze test

The water tank was filled with water enough to submerge the platform by 1 cm so that the platform was not visible from the mouse's field of view, and attached with a visual hint that the mouse could see around the water tank. The movements of the mice were captured with a camera attached to the ceiling and analyzed using the Any-maze system.

### Day 1 to 4: Trial

The mice were inserted at the designated location and allowed to explore for 1 minute to find the platform, and when the mice visited the platform within 1 minute, the experiment was terminated after confirming the mice stayed for 5 seconds.

When the mice failed to find the platform within 1 minute, the mice were induced to find the platform (Training, 10 seconds), and the above test was repeated a total of 3 times a day.

### Day 5: Probe test

After removing the platform, the mice were inserted at the designated location and allowed to explore for 1 minute, and the time spent in the target quadrant was compared in the probe test.

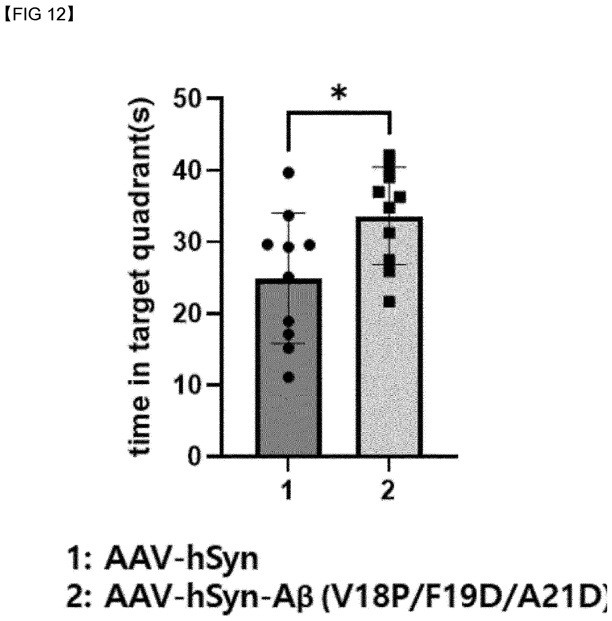

3xTg mice are Alzheimer's dementia model mice and have poor cognitive function and spatial perception. It was confirmed that as compared to 3xTg with reduced cognitive function, the mice transfected with the peptide according to the present invention by the AAV virus carrier, showed improvement in cognitive function, which is shown in FIG. 12.

There was no difference between groups in the time to find the platform according to the trial progress from Day 1 to Day 4. However, as could be confirmed in FIG. 12, the time spent in the target quadrant in the Day 5 probe test was longer in the group treated with pAAV-hSyn-GLSP-Aβ(V18P/F19D/A21D)-KK-WPRE-polyA than that of AAV-hSyn (Control). These results indicate that memory and spatial perception were improved (* : P < 0.05, ** : P <0.01).

From the above description, those knowledgeable in the field to which the present disclosure pertains will understand that the present disclosure may be embodied in other specific forms without changing the technical spirit or essential characteristics thereof. In this regard, it should be understood that the embodiments described above are illustrative in all respects and not restrictive. As the scope of the present disclosure, it should be construed that all changes or modifications derived from the meaning and scope of the claims to be described below and equivalents thereof rather than the above detailed description are included in the scope of the present disclosure.

## Claims

1. A genetic construct comprising: a coding sequence encoding an Aβ peptide variant and a promoter operably linked thereto.

2. The genetic construct of claim 1, wherein the coding sequence encoding the Aβ peptide variant is a sequence encoding a peptide sequence containing at least one mutation selected from the group consisting V18P, F19D, and A21D based on the Aβ42 peptide sequence of SEQ ID NO: 7.

3. The genetic construct of claim 2, wherein the coding sequence encoding the Aβ peptide variant is a sequence encoding a peptide sequence containing at least one mutation selected from the group consisting of V18P/A21D, and V18P/F19D/A21D based on the Aβ42 peptide sequence of SEQ ID NO: 7.

4. The genetic construct of claim 3, wherein the coding sequence encoding the Aβ peptide variant is SEQ ID NOs: 9 or 10.

5. The genetic construct of claim 3, wherein the Aβ peptide variant is SEQ ID NOs: 11 or 12.

6. The genetic construct of claim 1, wherein the promoter is an overexpression promoter or a neuron-specific promoter.

7. The genetic construct of claim 6, wherein the promoter is any one selected from the group consisting of a human synapsin I (SYN) promoter, a mouse calcium/calmodulin-dependent protein kinase II (CaMKII) promoter, a rat tubulin alpha I (Ta1) promoter, a rat neuron-specific enolase (NSE) promoter, a human platelet-derived growth factor-beta chain (PDGF) promoter, an EF-1α promoter, a CAG promoter and a CMV promoter.

8. The genetic construct of claim 7, wherein the promoter is a human synapsin I (SYN), CaMKII or CAG promoter, represented by SEQ ID NO: 17, 18 or 23, respectively.

9. The genetic construct of claim 1, further comprising at least one selected from the group consisting of an enhancer sequence, a polyadenylation sequence, and Kozak sequence.

10. A recombinant expression vector comprising the genetic construct according to any one of claims 1 to 9.

11. The recombinant expression vector of claim 10, wherein the recombinant expression vector is any one selected from the group consisting of an adenovirus vector, an adeno-associated virus (AAV) vector, a herpes virus vector, an avipoxvirus vector, and a lentivirus vector.

12. The recombinant expression vector of claim 11, wherein the recombinant expression vector is the adeno-associated virus (AAV) vector, which is any one selected from the group consisting of AAV1, AAV2, AAV3, AAV4, AAV5, AAV6, AAV7, AAV8, AAV9, AAV10, AAV11, AAV12, AAV13, AAV14, AAV15, AAV16, AAV.rh8, AAV.rh10, AAV.rh20, AAV.rh39, AAV.Rh74, AAV.RHM4-1, AAV.hu37, AAV.Anc80, AAV.Anc80L65, AAV.7m8, AAV.PHP.B, AAV.PHP.eB, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15 and AAV.HSC16.

13. The recombinant expression vector of claim 12, wherein the adeno-associated virus (AAV) vector is any one selected from the group consisting of AAV2, AAV7, AAV8, AAV9, AAV.rh8, AAV.rh10, AAV.rh20, AAV.rh39, AAV.Rh74, AAV.RHM4-1, AAV.hu37, AAV.PHP.B, AAV.PHP.eB and AAV.7m8.

14. A pharmaceutical composition for preventing or treating neurodegenerative diseases, comprising the genetic construct according to any one of claims 1 to 9.

15. A pharmaceutical composition for preventing or treating neurodegenerative diseases, comprising the recombinant expression vector according to claim 10.

16. The pharmaceutical composition of claim 14, wherein the neurodegenerative disease is any one or more selected from the group consisting of Alexander disease, Alpers disease, Alzheimer's disease, amyotrophic lateral sclerosis (ALS), ataxia-telangiectasia, neuronal ceroid lipofuscinoses, Batten disease, bovine spongiform encephalopathy (BSE), Canavan disease, cerebral palsy, Cockayne syndrome, corticobasal degeneration, Creutzfeldt-Jakob disease, frontotemporal lobe degeneration, Gaucher disease, Huntington's disease, HIV-associated dementia, Kennedy disease, Krabbe disease, Lewy body dementia, lysosomal storage disorder, neuroborreliosis, Machado-Joseph disease, motor neuron disease, multisystem atrophy, multiple sclerosis, multiple sulfatase deficiency, mucolipidosis, narcolepsy, Niemann-Pick type C, Niemann-Pick disease, Parkinson's disease, Pelizaeus-Merzbacher disease, Pick's disease, Pompe disease, primary lateral sclerosis, prion disease, progressive supranuclear palsy, Refsum disease, Sandhoff disease, Schilder disease, subacute combined degeneration of the spinal cord secondary to pernicious anemia, Spielmeyer-Vogt-Sjogren-Batten disease, spinocerebellar ataxia, spinal muscular atrophy, Steele Richardson Olszewski syndrome, spinal cord syphilis, and Tay-Sachs disease.

17. The pharmaceutical composition of claim 15, wherein the neurodegenerative disease is any one or more selected from the group consisting of Alexander disease, Alpers disease, Alzheimer's disease, amyotrophic lateral sclerosis (ALS), ataxia-telangiectasia, neuronal ceroid lipofuscinoses, Batten disease, bovine spongiform encephalopathy (BSE), Canavan disease, cerebral palsy, Cockayne syndrome, corticobasal degeneration, Creutzfeldt-Jakob disease, frontotemporal lobe degeneration, Gaucher disease, Huntington's disease, HIV-associated dementia, Kennedy disease, Krabbe disease, Lewy body dementia, lysosomal storage disorder, neuroborreliosis, Machado-Joseph disease, motor neuron disease, multisystem atrophy, multiple sclerosis, multiple sulfatase deficiency, mucolipidosis, narcolepsy, Niemann-Pick type C, Niemann-Pick disease, Parkinson's disease, Pelizaeus-Merzbacher disease, Pick's disease, Pompe disease, primary lateral sclerosis, prion disease, progressive supranuclear palsy, Refsum disease, Sandhoff disease, Schilder disease, subacute combined degeneration of the spinal cord secondary to pernicious anemia, Spielmeyer-Vogt-Sjogren-Batten disease, spinocerebellar ataxia, spinal muscular atrophy, Steele Richardson Olszewski syndrome, spinal cord syphilis, and Tay-Sachs disease.

18. Aβ peptide variant of SEQ ID NO: 11 or SEQ ID NO: 12.

19. A polynucleotide encoding the Aβ peptide according to claim 18.

20. A method for treating neurodegenerative diseases comprising: administering a therapeutically effective amount of the genetic construct according to any one of claims 1 to 9 to a subject in need thereof.

21. Use of the genetic construct according to any one of claims 1 to 9 in the manufacture of a medicament for use in the treatment of neurodegenerative diseases.

22. A pharmaceutical composition for use in preventing or treating neurodegenerative diseases, comprising the genetic construct according to any one of claims 1 to 9.
